# EUROPÄISCHE PATENTSCHRIFT

(11) **EP 1 904 127 B1**
(45) Veröffentlichungstag und Bekanntmachung des Hinweises auf die Patenterteilung: **10.08.2016**
(21) Anmeldenummer: 06754082.3
(22) Anmeldetag: 02.06.2006
(51) Int. Cl.: A61B 8/12, A61M 5/46, A61M 5/32, A61B 5/055

(54) **INJEKTIONSVORRICHTUNG UND -VERFAHREN**
INJECTION APPLIANCE AND METHOD
DISPOSITIF ET PROCEDE D'INJECTION

(30) Priorität: 03.06.2005 DE 102005025639
(43) Veröffentlichungstag der Anmeldung: 02.04.2008
(73) Patentinhaber: Innovacell Biotechnologie AG, 6020 Innsbruck (AT)
(72) Erfinder: MARKSTEINER, Rainer, A-6130 Schwaz (AT)
(74) Vertreter: Hertz, Oliver
(86) Internationale Anmeldenummer: PCT/EP2006/005286
(87) Internationale Veröffentlichungsnummer: WO 2006/128718

(56) Entgegenhaltungen:
- EP-A2- 0 956 873
- WO-A2-2006/002343
- WO-A2-2006/111861
- DE-A1- 10 134 911
- US-A- 3 063 449
- US-A- 4 737 151
- US-A- 4 892 520
- US-A- 4 900 303
- US-B1- 6 309 374

## Beschreibung

Die Erfindung betrifft eine Injektionsvorrichtung zur Injektion einer Substanz in einen Organismus mit den Merkmalen des Oberbegriffs von Anspruch 1, insbesondere eine Injektionsvorrichtung zur gezielten Injektion einer therapeutisch wirksamen Substanz an einem vorbestimmten Injektionsort im Gewebe des Organismus. Die Erfindung betrifft des Weiteren ein Injektionsverfahren unter Verwendung dieser Injektionsvorrichtung.

Aus EP 1 337 183 ist ein medizinisches Arbeitsgerät bekannt, mit dem eine Injektionsnadel in ein zu behandelndes Gewebe einführbar ist. Die Injektionsnadel wird an einem Austrittsende des Arbeitsgerätes mit einem Austrittswinkel relativ zu einer Achse des Arbeitsgerätes abgelenkt. Die Eindringtiefe der Injektionsnadel in das Gewebe hängt von der Austrittslänge der Spitze der Injektionsnadel relativ zum Austrittsende des Arbeitsgerätes ab. Bei der herkömmlichen Technik ist vorgesehen, die Positionierung der Injektionsnadel und die Injektion unter Verwendung einer Ultraschallsonde zu überwachen. Zur Vermeidung einer unerwünschten Verletzung des Gewebes ist bei der herkömmlichen Technik die Ultraschallsonde mit einem Anschlag ausgestattet, mit dem eine Verschiebung der Injektionsnadel aus dem Sichtfeld der Ultraschallsonde verhindert wird.

Mit der herkömmlichen Technik gemäß EP 1 337 183 wurde zwar ein erheblicher Fortschritt für die genaue Deposition von therapeutisch wirksamen Stoffen in ein Gewebe reicht. Nachteilig ist jedoch, dass eine grundsätzliche Verletzungsgefahr besteht, weil die Injektionsnadel zwar relativ zur Ultraschallsonde, nicht jedoch relativ zum Gewebe definiert positioniert werden kann. Bei der praktischen Anwendung des herkömmlichen Arbeitsgerätes können daher die folgenden Probleme auftreten. Wenn es auf eine Genauigkeit der Nadelpositionierung im mm- oder sub-mm-Bereich ankommt, kann der Gefahr einer Fehlpositionierung oder sogar einer Verletzung des umgebenden Gewebes nur durch eine besonders sorgfältige Beobachtung des Ultraschallbildes und eine langsame Vorschubbewegung der Injektionsnadel begegnet werden. Ein langsamer Injektionsprozess kann jedoch, insbesondere wenn in einem Organ an mehreren Injektionsorten Injektionen vorgenommen werden sollen, die Behandlung verzögern und zu einer unerwünschten Belastung der behandelten Person führen. Außerdem stellt die genaue Positionierung der Injektionsnadel besonders hohe Anforderungen an die Erfahrungen und das Geschick des behandelnden Arztes.

Das in EP 1 337 183 beschriebene Verfahren dient besonders der gezielten Injektion von Substanzen in die Wand von Körperhöhlen oder Röhren (z. B. der Harnröhre), wobei die Schallsonde zur Darstellung der Wände in die Injektionsvorrichtung integriert oder mit dieser verbunden ist. Diese Ausführung kann jedoch nachteilig sein, da die Schall- oder MRI-Sonde in den Körperhöhlen oder dem jeweiligen Körperlumen Platz beansprucht, wodurch es zu einer Dehnungsbeanspruchung des umliegenden Gewebes mit einer potentiellen Verletzungsgefahr kommen kann.

Aus US 6 309 374 B1 ist ein Injektionsgerät zur Injektion von Substanzen in Gewebe bekannt, bei dem eine Injektionsnadel auf einer Nadelführungsplattform beweglich ist. Die Nadelführungsplattform weist eine Stützoberfläche zum Aufsetzen des Injektionsgeräts auf das Gewebe auf. Zur Injektion wird die Nadel durch einen Kanal geführt, der in der Stützoberfläche mündet.

Weitere medizinische Arbeitsgeräte für Injektionszwecke sind aus DE 101 34 911 A1, EP 0 956 873 A2 und WO 2006/111861 A2 (Artikel 54(3) EPÜ) bekannt. Ein Gerät zur Applikation eines medizinischen Hilfswerkzeugs in biologisches Gewebe ist in WO 2006/002343 A2 (Artikel 54(3) EPÜ) beschrieben.

DE 101 34 911 A1 offenbart eine Ultraschallsonde mit einer Positioniereinrichtung für Untersuchungs- und Operationsvorrichtungen, wobei auf einem Schaft eine Führung für ein Injektionswerkzeug verschiebbar angeordnet ist. Aus EP 0 956 873 A2 ist ein Injektionsgerät mit einem Gehäuse bekannt, in dem ein Behältnis zur Aufnahme eines zu injizierenden Produkts und eine Injektionsnadel verschiebbar angeordnet sind.

Die Aufgabe der Erfindung ist es, ein verbessertes medizinisches Arbeitsgerät zu schaffen, mit dem die Nachteile der herkömmlichen Technik überwunden werden und das insbesondere die Gefahr von Fehlpositionierungen und/oder Verletzungen vermindert und eine Beschleunigung einer Injektionsbehandlung ermöglicht.

Diese Aufgabe wird mit einer Injektionsvorrichtung mit den Merkmalen von Patentanspruch 1 gelöst. Vorteilhafte Ausführungsformen und Anwendungen der Erfindung ergeben sich aus den abhängigen Ansprüchen.

Vorrichtungsbezogen basiert die Erfindung auf der allgemeinen technischen Lehre, eine Injektionsvorrichtung zur Injektion einer Substanz in einen Organismus mit einer Verschiebeeinrichtung, mit der ein Injektionswerkzeug verbunden ist und einer Führungseinrichtung mit einem Lumen zur Aufnahme des Injektionswerkzeuges bereitzustellen, wobei die Verschiebeeinrichtung und die Führungseinrichtung relativ zueinander verschiebbar mit einer Halteeinrichtung verbunden sind. Die Halteeinrichtung bildet einen gemeinsamen Träger für die Führungs- und Verschiebeeinrichtungen, der ortsfest relativ zum Organismus und insbesondere zum gewünschten Injektionsort positionierbar ist. Das Injektionswerkzeug ist vorzugsweise mit der Verschiebeeinrichtung fest verbunden, insbesondere an der Verschiebeeinrichtung befestigt. Vorteilhafterweise ermöglicht die Halteeinrichtung eine zuverlässige und genaue Einstellung der Führungs- und Verschiebeeinrichtungen relativ zum Organismus und relativ zueinander, so dass mit der Position beider voneinander unabhängig einstellbarer Teile der Injektionsort im Organismus genau festgelegt ist. Durch die Einstellung der Führungs- und Verschiebeeinrichtungen an der Halteeinrichtung wird die Gefahr einer Fehlpositionierung im Organismus vermieden.

Die Führungseinrichtung ist ein langgestrecktes Bauteil, z.B. Rohr oder Stab, in dessen Wand zumindest über einen Teil der Länge der Führungseinrichtung das Lumen zur Aufnahme des Injektionswerkzeuges verläuft. Die Führungseinrichtung erstreckt sich entlang einer Bezugslinie, die in Abhängigkeit von der gewünschten Anwendung der Injektionsvorrichtung gerade oder gekrümmt sein kann. Im ersten Fall weist die Führungseinrichtung die Form eines geraden Stabes oder Rohres auf, wobei die Bezugslinie einer Längsachse der Führungseinrichtung entspricht, während im zweiten Fall die Form eines gekrümmten Stabes oder Rohres vorgesehen ist, wobei die Bezugslinie einer Mittellinie der Führungseinrichtung entspricht. An einem freien, von der Halteeinrichtung wegweisenden Ende der Führungseinrichtung ist ein Austrittsende vorgesehen, an dem das Lumen endet. Am Austrittsende weist das Lumen einen vorbestimmten Winkel relativ zu der am Austrittsende gerade verlängerten Bezugslinie auf, welche die Form der Führungseinrichtung repräsentiert. Im Fall einer geraden Führungseinrichtung bildet das Lumen am Austrittsende einen Winkel relativ zur Bezugslinie (Längsachse) der Führungseinrichtung. Bei einer gekrümmten Form bildet das Lumen am Austrittsende einen Winkel mit der am Austrittsende gerade verlängerten Mittellinie der Führungseinrichtung.

Die Führungseinrichtung kann zumindest in dem Teilbereich, in dem das Lumen verläuft, aus einem starren Material, z. B. Metall (z. B. Edelstahl) oder Kunststoff (z. B. glasfaserverstärkte Kunststoffe, PVC, PEEK o. dgl.), oder aus einem flexiblen, insbesondere elastisch biegsamen Material, z. B. Kunststoff (z. B. PMMA) hergestellt sein.

Mit der Verschiebeeinrichtung kann das Injektionswerkzeug in dem Lumen der Führungseinrichtung verschoben werden. Die Verschiebeeinrichtung dient der Betätigung des Injektionswerkzeugs, wobei insbesondere die folgenden Betriebspositionen der Verschiebeeinrichtung an der Halteeinrichtung vorgesehen sind. In einer rückgezogenen Betriebsposition der Verschiebeeinrichtung ist das Injektionswerkzeug zur Halteeinrichtung zurückgezogen. In einer vorgeschobenen Betriebsposition der Verschiebeeinrichtung ist das Injektionswerkzeug vorgeschoben, so dass sich sein freies Ende von der Halteeinrichtung entfernt und zum Organismus hin bewegt. Die Verschiebeeinrichtung ist auf einem Verschiebeweg zwischen den Betriebspositionen verschiebbar. Bei der Führungseinrichtung sind ebenfalls insbesondere die folgenden Betriebspositionen vorgesehen. In einer rückgezogenen Betriebsposition der Führungseinrichtung ist diese zur Halteeinrichtung hin zurückgezogen, so dass das Austrittsende der Führungseinrichtung einen minimalen Abstand von der Halteeinrichtung aufweist. Umgekehrt weist das Austrittsende in einer vorgeschobenen Betriebsposition der Führungseinrichtung einen maximalen Abstand von der Halteeinrichtung auf. Die Führungseinrichtung ist auf einem Verschiebeweg zwischen den genannten Betriebspositionen verschiebbar und fixierbar.

Die Einstellung der Führungs- und Verschiebeeinrichtungen zwischen den genannten Betriebspositionen erfolgt in Abhängigkeit von der aktuellen Arbeitsphase der Injektionsvorrichtung und der gewünschten Einstichtiefe des Injektionswerkzeugs in ein Gewebe des Organismus. Die Länge des Injektionswerkzeuges, die Länge der Führungseinrichtung und die Verschiebewege der Führungs- und Verschiebeeinrichtungen auf der Halteeinrichtung sind so gewählt, dass in der rückgezogenen Betriebsposition der Verschiebeeinrichtung das Injektionswerkzeug komplett in das Lumen der Führungseinrichtung zurückgezogen ist. Dies ermöglicht vorteilhafterweise, dass die Führungseinrichtung ohne Verletzungsgefahr in einen zu behandelnden Organismus, z.B. in ein Hohlorgan des Organismus einführbar ist.

Die Einstellung der Führungseinrichtung zwischen den rückgezogenen und vorgeschobenen Betriebspositionen legt die Position des Austrittsendes der Führungseinrichtung relativ zur Verschiebeeinrichtung und damit eine freie Austrittslänge des Injektionswerkzeugs in einer vorgeschobenen Betriebsposition der Verschiebeeinrichtung fest. Durch die Bildung eines Winkels zwischen dem Ende des Lumens und der Bezugslinie der Führungseinrichtung wird durch die Austrittslänge des Injektionswerkzeugs gleichzeitig eine Einstichtiefe in das Gewebe des Organismus definiert, wie dies in EP 1 337 183 beschrieben ist. Wenn sich die Führungseinrichtung in einer zurückgezogenen Betriebsposition befindet, wird in der vorgeschobenen Betriebsposition der Verschiebeeinrichtung eine größere Austrittslänge des Injektionswerkzeugs und damit eine größere Einstichtiefe erzielt, als wenn sich die Führungseinrichtung in einer vorgeschobenen Betriebsposition befindet.

Die erfindungsgemäße Injektionsvorrichtung weist den besonderen Vorteil auf, dass durch die Einstellung der Führungs- und Verschiebeeinrichtungen relativ zueinander an dem gemeinsamen Träger in vorbestimmter Weise die Einstichtiefe des Injektionswerkzeugs festgelegt werden kann.

Gemäß einer bevorzugten Ausführungsform der Erfindung weist die Halteeinrichtung einen Vorschub-Anschlag auf, durch den die Vorschubbewegung der Verschiebeeinrichtung bis zu einem Maximalwert begrenzt wird. Die Position des Vorschub-Anschlags ist so gewählt, dass selbst bei einer zurückgezogenen Führungseinrichtung das Injektionswerkzeug nicht tiefer als eine vorbestimmte Grenztiefe in das Gewebe einsticht. Vorteilhafterweise kann damit eine unbeabsichtigte Verletzung benachbarten Gewebes ausgeschlossen werden. Im Unterschied zum herkömmlichen medizinischen Arbeitsgerät, bei dem ein Anschlag des Injektionswerkzeugs relativ zu einer Ultraschallsonde oder zu deren Schaft vorgesehen war und damit nur eine Fehlpositionierung außerhalb des Sichtfeldes der Ultraschallsonde vermieden werden konnte, ermöglicht der Vorschub-Anschlag der Halteeinrichtung eine Begrenzung der Bewegung des Injektionswerkzeugs im ortsfesten Koordinatensystem der Halteeinrichtung. Damit werden die folgenden wichtigen Vorteile der erfindungsgemäßen Injektionsvorrichtung erreicht. Erstens werden unbeabsichtigte Verletzungen umgebenden Gewebes ausgeschlossen. Zweitens kann der Vorschub des Injektionswerkzeugs zum Injektionsort mit einer relativ hohen Geschwindigkeit erfolgen, da ein unbeabsichtigtes Verfehlen des Injektionsortes durch den Vorschub-Anschlag ausgeschlossen ist. Die Injektion in den Organismus wird beschleunigt, was sich besonders bei der Injektion an einer Vielzahl benachbarter Injektionsorte, wie z.B. bei der Injektion in die Wand der Harnröhre auswirkt.

Gemäß einer weiteren, bevorzugten Ausführungsform der Erfindung weist die Halteeinrichtung einen Rückzug-Anschlag auf, durch den die Rückzugsbewegung der Führungseinrichtung relativ zur Halteeinrichtung begrenzt wird. Vorteilhafterweise kann durch den Rückzug-Anschlag ebenfalls die maximale Einstichtiefe des Injektionswerkzeugs und zusätzlich auch der sichere Rückzug des Injektionswerkzeugs in das Lumen in der rückgezogenen Betriebsposition der Verschiebeeinrichtung sichergestellt werden. Die Position des Rückzug-Anschlags wird somit vorzugsweise so gewählt, dass sich das Injektionswerkzeug in der rückgezogenen Betriebsposition der Verschiebeeinrichtung komplett im Lumen der Führungseinrichtung befindet.

Die Führungs- und Verschiebeeinrichtungen sind an der Halteeinrichtung gerade verschiebbar angeordnet. Hierzu kann grundsätzlich jede an sich verfügbare Linearführung realisiert werden, die beispielsweise auf der Oberfläche der Halteeinrichtung angeordnet ist. Gemäß einer bevorzugten Ausführungsform der Erfindung weist die Halteeinrichtung jedoch einen rohrförmigen Aufbau auf, in dessen Innenraum die Führungseinrichtung und auf dessen Oberfläche die Verschiebeeinrichtung angeordnet sind. In der Wand des rohrförmigen Aufbaus sind Ausnehmungen zur Durchführung des Injektionswerkzeugs von der Verschiebeeinrichtung in das Lumen der Führungseinrichtung und ggf. für mindestens einen der Vorschub- und Rückzug-Anschläge oder ggf. weitere Stelleinrichtungen vorgesehen. Die rohrförmige Halteeinrichtung ermöglicht vorteilhafterweise einen kompakten Aufbau der Injektionsvorrichtung, da die Führungs- und Verschiebeeinrichtungen zumindest in Teilbereichen koaxial angeordnet sein können. Des weiteren ermöglicht der rohrförmige Aufbau bei einer geraden Form eine Drehbarkeit der Führungs- und Verschiebeeinrichtungen um eine Längsachse der Halteeinrichtung. Wenn die Führungs- und Verschiebeeinrichtungen gemäß einer bevorzugten Variante der Erfindung entsprechend drehbar angeordnet sind, können sich Vorteile für eine allseitige Injektion durch die Wand eines Hohlorgans, wie z.B. der Harnröhre ergeben.

Erfindungsgemäß ist die Injektionsvorrichtung mit einem Drehlager ausgestattet, in dem die Halteeinrichtung angeordnet ist. Das Drehlager mit einem Haltebolzen ist ortsfest relativ zum zu behandelnden Organismus positionierbar. Die im Drehlager sitzende Halteeinrichtung ist relativ zu dem Organismus nicht verschiebbar. Der Aufbau der Halteeinrichtung mit den Führungs- und Verschiebeeinrichtungen ist in dem Drehlager verdrehbar angeordnet. Das Drehlager ist gemäß der Erfindung mit einem Dorn ausgestattet, der eine Verdrehung der Halteeinrichtung in der vorgeschobenen Betriebsposition der Verschiebeeinrichtung blockiert, so dass vorteilhafterweise eine unbeabsichtigte Drehung, während das Injektionswerkzeug aus der Führungseinrichtung herausragt, und damit eine Verletzung des Organismus ausgeschlossen werden. Gemäß der Erfindung weist die Verschiebeeinrichtung einen Lochkranz auf, der mit dem Dorn des Drehlagers zusammenwirkt. Der Lochkranz enthält vorzugsweise mehrere Führungslöcher, die einen Vorschub der Verschiebeeinrichtung entsprechend vorbestimmten Winkelpositionen ermöglicht.

Vorteilhafterweise die Anzahl und/oder die Winkelverteilung der Führungslöcher in Abhängigkeit von der gewünschten geometrischen Verteilung von Injektionsorten im Organismus gewählt werden. Mit dem Lochkranz wird somit eine Maske vorgegeben, die eine zuverlässige, radial vollständige Injektion in der Wand eines Hohlorgans ermöglicht.

Weitere Vorteile für die Zuverlässigkeit und Reproduzierbarkeit der Wahl des Injektionsortes ergeben sich, da erfindungsgemäß die Bewegung der Führungseinrichtung relativ zur Halteeinrichtung blockierbar ist. Es ist eine lösbare Fixierung der Führungseinrichtung an der Halteeinrichtung vorgesehen. Erfindungsgemäß wird hierzu eine erste Klemmeinrichtung verwendet, mit der die Position der Führungseinrichtung an der Halteeinrichtung zwischen den vorgeschobenen und rückgezogenen Betriebspositionen feststellbar ist. Wenn als erste Klemmeinrichtung eine Klemmschraube verwendet wird, die von außen durch einen Schlitz in der Wand der rohrförmigen Halteeinrichtung in den Körper der Führungseinrichtung eingesetzt ist, können sich Vorteile für eine vereinfachte Bedienung und Verstellung der Führungseinrichtung ergeben. Besonders bevorzugt ist die Klemmschraube mit einem inneren Spülkanal ausgestattet, durch den eine Spülflüssigkeit in einen Kanal in der Führungseinrichtung einführbar ist.

Bei der praktischen Anwendung der erfindungsgemäßen Injektionsvorrichtung ist diese vorzugsweise mit einer bildgebenden Sonde, wie z.B. einer Ultraschallsonde oder einer MRI-Sonde ausgestattet, um die Bewegung des Injektionswerkzeugs in den Organismus und/oder die Injektion einer Substanz am Injektionsort zu überwachen. Vorzugsweise ist die Führungseinrichtung mit einem inneren Kanal ausgestattet, in dem die bildgebende Sonde angeordnet werden kann, so dass das aktive Teil, z.B. der Ultraschallwandler, am Austrittsende der Führungseinrichtung aus dem Kanal herausragt. Die bildgebende Sonde kann mit der Führungseinrichtung fest verbunden sein. Vorteile in Bezug auf die Flexibilität der Anwendung der Injektionsvorrichtung ergeben sich jedoch, wenn die bildgebende Sonde im Kanal der Führungseinrichtung verschiebbar angeordnet ist, um an die aktuelle Position der Führungseinrichtung und der Verschiebeeinrichtung angepasst werden zu können. Wenn gemäß einer bevorzugten Variante eine zweite Klemmeinrichtung vorgesehen ist, mit der die Sonde relativ zur Führungseinrichtung fixierbar ist, ergeben sich Vorteile für die Zuverlässigkeit der Überwachung der Injektion.

Erfindungsgemäß kann die Verschiebeeinrichtung nicht nur zur Betätigung des Injektionswerkzeugs, sondern auch der Aufnahme eines Reservoirs für eine zu injizierende Substanz dienen. Hierzu kann gemäß einer weiteren Ausführungsform der Erfindung die Verschiebeeinrichtung mit einer Halterung ausgestattet sein, in die das Reservoir, wie z.B. der Kolben einer Spritzennadel einsetzbar ist. Besonders bevorzugt ist die Halterung mit einer Stellschraube zur Dosierung der zu injizierenden Substanz ausgestattet, wodurch die Menge der Injektion genau und reproduzierbar einstellbar ist.

Gemäß einem weiteren wichtigen Merkmal der Erfindung kann die Injektionsvorrichtung mit einer Sensoreinrichtung ausgestattet sein, die einen oder mehrere Sensoren zur Erfassung der Position von wenigstens einer der Führungs- Verschiebe- und Halteeinrichtungen aufweist. Vorteilhafterweise können mit der Sensoreinrichtung Signale für eine automatisierte Einstellung der relativ zueinander verschiebbaren Teile der Injektionsvorrichtung in Abhängigkeit von einer vorgegebenen Injektionsaufgabe bereitgestellt werden.

Die Betriebssicherheit der erfindungsgemäßen Injektionsvorrichtung kann vorteilhafterweise weiter gesteigert werden, wenn die Halteeinrichtung mit einer Rückzugsfeder ausgestattet ist, die bei freier Beweglichkeit der Verschiebeeinrichtung deren Rückzug in die rückgezogene Betriebsposition bewirkt. Vorteilhafterweise wird insbesondere bei einer manuell betriebenen Injektionsvorrichtung die Verschiebeeinrichtung gegen die Wirkung der Rückzugsfeder vorgeschoben, bis das Injektionswerkzeug aus dem Lumen der Führungseinrichtung herausragt. Wenn diese manuelle Vorschubkraft wegfällt, bewirkt die Rückzugsfeder automatisch den Rückzug der Verschiebeeinrichtung und damit den Rückzug des Injektionswerkzeuges in das Lumen.

Die erfindungsgemäße Injektionsvorrichtung wird derart verwendet, dass zunächst die Führungseinrichtung in dem Organismus positioniert und anschließend die Verschiebeeinrichtung zum Vorschub des Injektionswerkzeugs betätigt wird. Beispielsweise erfolgt mit der Positionierung der Führungseinrichtung eine Positionierung einer bildgebenden Sonde, die eine Bildaufnahme des gewünschten Injektionsortes ermöglicht. Alternativ kann die Positionierung der bildgebenden Sonde unabhängig von der Injektionsvorrichtung erfolgen, wobei sich Vorteile für eine kompaktere Bauform der Injektionsvorrichtung und eine verminderte Dehnungsbeanspruchung des umliegenden Gewebes ergeben können.

Wenn die Betätigung der Verschiebeeinrichtung zur Einführung des Injektionswerkzeuges bis zum Injektionsort einen Vorschub bis zum Vorschub-Anschlag umfasst, kann die Injektion vorteilhafterweise beschleunigt werden.

Wenn die Injektion gemäß einer Variante des genannten Verfahrens unter Verwendung eines Spritzenkolbens erfolgt, kann die Dosierung der zu injizierenden Substanz erleichtert werden.

Weitere Einzelheiten und Vorteile der Erfindung werden aus der Beschreibung der beigefügten Zeichnungen ersichtlich. Es zeigen:
- Figuren 1 bis 3:: schematische Schnittansichten verschiedener Betriebszustände einer Injektionsvorrichtung gemäß einer ersten Ausführungsform der Erfindung;
- Figur 4:: eine schematische Schnittansicht einer Injektionsvorrichtung gemäß einer weiteren Ausführungsform der Erfindung;
- Figuren 5A und 5B: eine Schnittansicht und eine Draufsicht auf eine Injektionsvorrichtung gemäß einer weiteren Ausführungsform der Erfindung; und
- Figuren 6 bis 9:: schematische Ansichten von Einzelteilen der in Figur 5 gezeigten Injektionsvorrichtung.

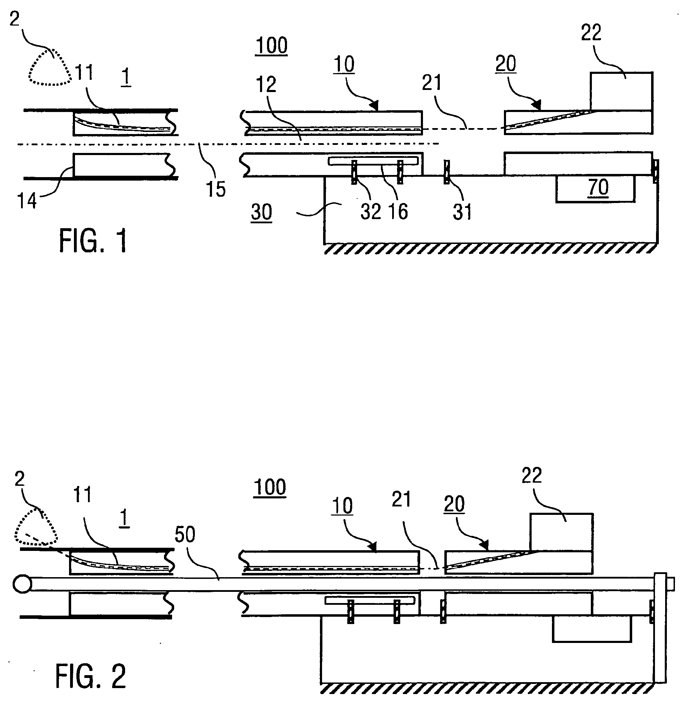

Figur 1 illustriert eine erste Ausführungsform der erfindungsgemäßen Injektionsvorrichtung 100 in schematischer Schnittansicht. Die Injektionsvorrichtung 100 umfasst die Führungseinrichtung 10, die Verschiebeeinrichtung 20 und die Halteeinrichtung 30. Das Bezugszeichen 70 in Figur 1 verweist schematisch auf eine Sensoreinrichtung, mit der Positionen der Führungseinrichtung 10 und der Verschiebeeinrichtung 20 relativ zur Halteeinrichtung 30 erfassbar sind. Die Sensoreinrichtung 70 umfasst beispielsweise optische Sensoren, mit denen Strichmarkierungen an den Führungs- und/oder Verschiebeeinrichtungen erfassbar sind.

Die Führungseinrichtung 10 ist ein gerades, starres Rohr mit einem Lumen 11 und einem Kanal 12. Das Rohr hat z. B. eine Halbzylinderform mit einer ebenen Seite. Das Lumen 11 ist zur Aufnahme des Injektionswerkzeugs 21 (gestrichelt gezeichnet) vorgesehen und am Austrittsende 14 der Führungseinrichtung 10 relativ zur Mittellinie 15 der Führungseinrichtung 10 abgewinkelt. Der Kanal 12 ist bei der dargestellten Ausführungsform zur Aufnahme einer bildgebenden Sonde vorgesehen (in Figur 2 dargestellt). Die Länge der Führungseinrichtung 10 beträgt z. B. rund 40 cm. Aus Klarheitsgründen ist in Figur 1 nicht die gesamte Länge der Führungseinrichtung 10 dargestellt, sondern mit der geschwungenen Linie eine Unterbrechung illustriert. Die Führungseinrichtung 10 ist mit einer Linearführung 16 (schematisch dargestellt) auf der Halteeinrichtung 30 verschiebbar angeordnet.

Das Injektionswerkzeug 21 ist eine Injektionsnadel, die an der Verschiebeeinrichtung 20 befestigt ist. Die Verschiebeeinrichtung 20 trägt auch eine Aufnahme 22 für ein Injektionsreservoir (nicht dargestellt), von dem die zu injizierende Substanz durch das Injektionswerkzeug 21 zum Injektionsort im Gewebe einführbar ist. Die Verschiebeeinrichtung 20 ist wie ein Schlitten ebenfalls mit einer Linearführung (nicht dargestellt) auf der Halteeinrichtung 30 geradlinig verschiebbar angeordnet.

Die Halteeinrichtung 30 ist mit einem Vorschub-Anschlag 31 ausgestattet, mit dem die Vorschubbewegung der Verschiebeeinrichtung 20 begrenzt wird. Des weiteren ist für die Führungseinrichtung 10 ein Rückzugs-Anschlag 32 vorgesehen, mit dem die Rückzugsbewegung der Führungseinrichtung 10 begrenzt wird. Wenn die Verschiebeeinrichtung 20 am Vorschub-Anschlag 31 und die Führungseinrichtung 10 am Rückzug-Anschlag 32 anliegen, kann das Injektionswerkzeug 21 nur bis zu einer vorgegebenen Maximaltiefe in das Gewebe des Organismus 1 hineinragen. Eine Verletzung des benachbarten Gewebes ist ausgeschlossen.

Figur 1 zeigt die Injektionsvorrichtung 100 in einem Betriebszustand, in dem die Führungseinrichtung in ein Hohlorgan, z.B. die Harnröhre eines zu behandelnden Patienten-Organismus 1 eingeführt ist. Die Führungseinrichtung 10 ist so positioniert, dass das Austrittsende 14 einen vorbestimmten Abstand zu dem gewünschten Injektionsort 2 in der Wand der Harnröhre aufweist. Dieser Abstand ist insbesondere in Abhängigkeit von der gewünschten Injektionstiefe gewählt, wie dies unten illustriert ist. In dem in Figur 1 gezeigten Betriebszustand befindet sich die Verschiebeeinrichtung 20 in der rückgezogenen Betriebsposition, so dass das Injektionswerkzeug 21 nicht aus dem Lumen 11 herausragt.

Die Positionierung der Führungseinrichtung 10 im Organismus 1 gemäß Figur 1 erfolgt unter Beobachtung mit einer bildgebenden Sonde 50, die aus Übersichtlichkeitsgründen in Figur 1 nicht dargestellt ist. Die Sonde 50 ist an der Halteeinrichtung 30 lösbar fixiert (siehe Figur 2). Sie wird von den Führungs- und Verschiebeeinrichtungen gehalten, die relativ zur Sonde 50 verschiebbar sind.

In Figur 2 ist der Betriebszustand der Injektionsvorrichtung 100 mit vorgeschobenen Injektionswerkzeug 21 illustriert. Durch die Neigung des Lumens 11 relativ zur Längsachse der Führungseinrichtung 10 ragt das Ende des Injektionswerkzeugs 21 in das Gewebe 2. In diesem Zustand wird ein an der Halterung 22 angebrachtes Injektionsreservoir betätigt, um eine Substanz in das Gewebe 2 zu injizieren.

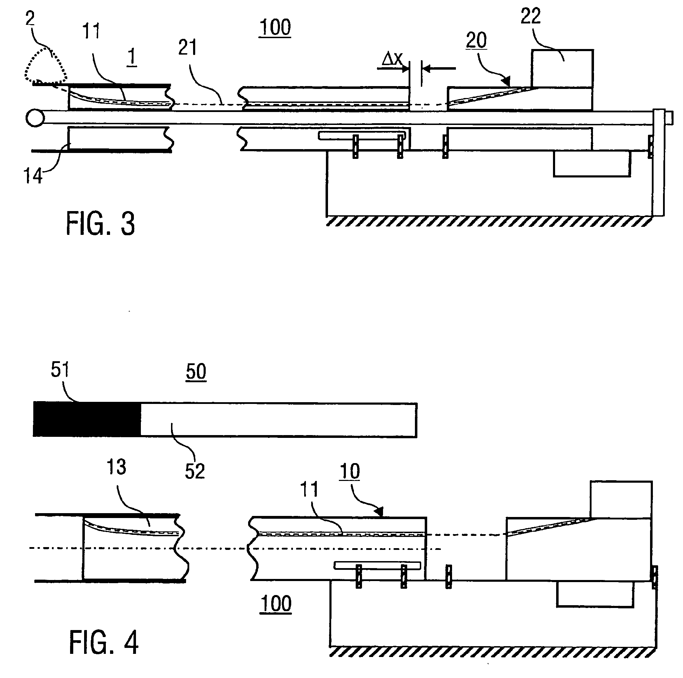

Figur 3 zeigt wie Figur 2 einen Betriebszustand der Injektionsvorrichtung 100, bei der das Injektionswerkzeug 21 in das Gewebe 2 vorgeschoben ist. In diesem Fall ist jedoch auch die Führungseinrichtung 10 um einen Abstand Δx vorgeschoben, so dass der Abstand des Austrittsendes 14 vom Gewebe 2 verringert und damit die Eindringtiefe des Injektionswerkzeugs 21 in das Gewebe 2 vermindert ist.

Zur Umsetzung der Erfindung in der Praxis ist es nicht notwendig, dass die bildgebende Sonde 50 im Kanal 12 angeordnet ist. Alternativ kann die bildgebende Sonde 50 (zum Beispiel Ultraschall- oder MRI-Sonde) getrennt von der Injektionsvorrichtung auf der Körperoberfläche (z. B. zur Aufnahme von dreidimensionalen Bildern) oder in einer benachbarten Körperhöhle angeordnet sein. Für diesen Fall kann der Kanal 12 in der Führungseinrichtung 10 weggelassen werden Die Führungseinrichtung 10 ist dann ein gerades, starres stabförmiges Bauteil 13, das nur das Lumen 11 enthält und im übrigen kompakt ist, wobei die bildgebende Sonde 50 mit einem Schallkopf 51 und einem Schaft 52 separat ausgerichtet werden, wie dies in Figur 4 gezeigt ist.

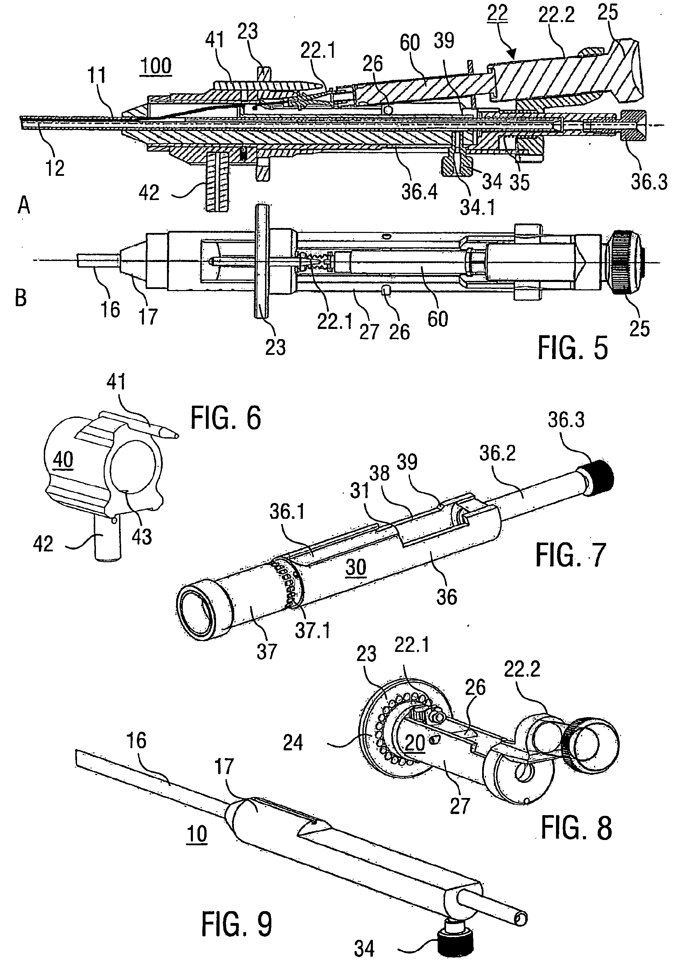

Die Figuren 5A, 5B illustrieren eine abgewandelte Ausführungsform der erfindungsgemäßen Injektionsvorrichtung 100, die wegen der kompakten Bauform des koaxialen Aufbaus der Führungs-, Verschiebe- und Halteeinrichtungen bevorzugt realisiert wird.

In dem in den Figuren 5A, 5B gezeigten Betriebszustand befindet sich die Verschiebeeinrichtung 20 in der vorgeschobenen Betriebsposition. Die Schnittdarstellung gemäß Figur 5A und die Perspektivansicht gemäß Figur 9 zeigen die Führungseinrichtung 10 mit einem geraden Formrohr 16, in dem das Lumen 11 und der Kanal 12 verlaufen. Der Kanal 12 ist zur Aufnahme der bildgebenden Sonde (nicht dargestellt) vorgesehen. Aus Übersichtlichkeitsgründen ist nicht die gesamte Länge des Formrohres 16 gezeigt. Des weiteren umfasst die Führungseinrichtung 10 einen Führungskörper 17, dessen äußerer Durchmesser an den Innendurchmesser der rohrförmigen Halteeinrichtung 30 angepasst ist.

Die Verschiebeeinrichtung 20 (siehe auch Figur 8) umfasst ein Schlittenteil 27 in Form eines angeschnittenen Hohlzylinders mit einer auf seiner Oberseite geöffneten Mantelfläche und einem Querstift 26, der in einer Linearführung 38 der Halteeinrichtung 30 aufliegt. Die Verschiebeeinrichtung 20, mit der das Injektionswerkzeug 21 verbunden ist, umfasst des Weiteren die Aufnahme 22 für das Injektionsreservoir 60 und einen Lochkranz 23 mit Führungslöchern 24. Zum Einsetzen des Injektionsreservoirs 60 in Form eines Spritzenkolbens umfasst die Halterung 22 einen Spritzenansatz 22.1 und einen Kolbenansatz 22.2, der mit einer Stellschraube 25 verstellbar ist. Der Innendurchmesser des Schlittenteils 27 entspricht dem Außendurchmesser des Zylinderteils 36 der Halteeinrichtung 30 (siehe unten). Das Schlittenteil 27 ist auf dem Zylinderteil 36 verschiebbar.

Die Halteeinrichtung 30 (siehe auch Figur 7) umfasst ein Zylinderteil 36 mit einem Lagerteil 37 und einer Führungshülse 36.2. Die Oberfläche des Zylinderteil 36 ist auf einer Oberseite aufgeschnitten, um die Linearführung 38 mit einem Verschiebeweg für die Verschiebeeinrichtung 20 bereitzustellen. Der Verschiebeweg verläuft zwischen dem Vorschub-Anschlag 31 und einem rückseitigen Anschlag 39. Des weiteren weist die Oberfläche des Zylinderteils 36 eine Ausnehmung 36.1 zur Durchführung des Injektionswerkzeugs 21 zur Führungseinrichtung 10 auf. Am rückseitigen Ende der Führungshülse 36.2 ist eine Klemmschraube 36.3 vorgesehen, mit der die bildgebende Sonde im Kanal 12 der Führungseinrichtung 10 fixierbar ist. Des Weiteren bildet die Führungshülse 36.2 einen Träger für eine Rückzugsfeder 35, die zwischen dem rückseitigen Ende des Zylinderteils 36 und dem rückseitigen Ende des Schlittenteils 27 angeordnet ist. In der vorgeschobenen Betriebsposition der Verschiebeeinrichtung ist die Rückzugsfeder 35 unter Zug gespannt.

Das Lagerteil 37 sitzt im Drehlager 40 (siehe Figur 6), das einen Dorn 41 aufweist. Der Dorn 41 wirkt mit dem Lochkranz 23 der Verschiebeeinrichtung 20 zusammen. Des weiteren ist das Drehlager 40 fest mit einem Haltebolzen 42 zur ortsfesten Positionierung z. B. auf einem Stativ (nicht dargestellt) ausgestattet. Im Drehlager 40 befindet sich eine Rastnase, z. B. eine federnd gelagerte Kugel 43, die mit Ausnehmungen 37.1 auf der Oberfläche des Lagerteils 37 zusammenwirkt.

Das Zylinderteil 36 der Halteeinrichtung 30 weist auf seiner Unterseite eine schlitzförmige Öffnung 36.4 auf (siehe Figur 5A), durch welche der Verschiebeweg der Führungseinrichtung 10 definiert wird. Zur zeitweiligen Fixierung der Führungseinrichtung 10 relativ zur Halteeinrichtung 30 ist eine Klemmschraube 34 mit einem inneren Spülkanal 34.1 vorgesehen, durch den ein Schlauch zur Einführung einer Spülflüssigkeit in den Kanal 12 der Führungseinrichtung 10 einlegbar ist.

Die Injektion unter Verwendung der erfindungsgemäßen Injektionsvorrichtung 100 erfolgt nach dem folgenden Verfahren. Zunächst wird die Halteeinrichtung 30 mit dem Drehlager 40 ortsfest relativ zum Organismus 1, z.B. zur Harnröhre eines Patienten positioniert. Das Formrohr 16 wird in die Harnröhre eingeführt, wobei die Einführung in der rückgezogenen Betriebsposition der Verschiebeeinrichtung 20 (Querstift 26 am rückseitigen Anschlag 39) und unter Beobachtung mit der Ultraschallsonde 50 erfolgt. Die Führungseinrichtung 10 wird mit einem Abstand des Austrittsendes 14 (siehe Figur 1) vom Gewebe 2 so positioniert, dass bei vorgeschobener Betriebsposition der Verschiebeeinrichtung 20 (Querstift 26 am Vorschub-Anschlag 31) die Injektionsnadel in der vorgeschobenen Position der Verschiebeeinrichtung 20 den gewünschten Injektionsort im Gewebe 2 treffen würde. Anschließend erfolgt die manuelle Bewegung der Verschiebeeinrichtung 20, so dass das Injektionswerkzeug 21 vorgeschoben wird. Während der behandelnde Arzt mit einer Hand z. B. den Haltebolzen 42 des Drehlagers 40 und den Lochkranz 23 umgreift, kann mit der anderen Hand die Stellschraube 25 zum Vorschub des Spritzenkolbens 62 in der Spritze 61 betätigt werden. Nach Injektion einer vorbestimmten Menge, die z. B. durch eine bestimmte Anzahl von Umdrehungen der Stellschraube 25 repräsentiert wird, wird die Verschiebeeinrichtung 20 freigegeben. Daraufhin wird die Verschiebeeinrichtung 20 unter der Wirkung der Rückzugsfeder 35 zurückgezogen, bis sich der Lochkranz 23 von dem Dorn 41 trennt. In dieser Situation kann die Halteeinrichtung 30 mit der Führungseinrichtung 10 und der Verschiebeeinrichtung 20 um ein Führungsloch 24 weitergedreht und die Verschiebeeinrichtung 20 erneut bis zum Vorschub-Anschlag 31 vorgeschoben werden. In dieser Position erfolgt die nächste Injektion. Dieser Vorgang wird wiederholt, bis eine komplette Umdrehung absolviert ist. Anschließend wird die Halteeinrichtung 30 z. B. durch eine Verschiebung des Drehlagers 40 am Stativ zurückgezogen, um eine weitere radiale Injektion in der Harnröhre zu setzen.

Die in der vorstehenden Beschreibung, den Zeichnungen und den Ansprüchen offenbarten Merkmalen der Erfindung können sowohl einzeln als auch in beliebiger Kombination für die Verwirklichung der Erfindung in ihren verschiedenen Ausgestaltungen von Bedeutung sein.

## Patentansprüche

1. Injektionsvorrichtung (100) zur Injektion einer Substanz in einen Organismus (1), umfassend:
- eine Führungseinrichtung (10) mit einem Lumen (11), in dem ein Injektionswerkzeug (21) verschiebbar angeordnet ist,
- eine Verschiebeeinrichtung (20), mit der das Injektionswerkzeug (21) verbunden ist, und
- eine Halteeinrichtung (30), die einen Träger für die Führungseinrichtung (10) und für die Verschiebeeinrichtung (20) bildet, wobei die Führungs- und Verschiebeeinrichtungen (10, 20) relativ zueinander und relativ zu der Halteeinrichtung (30) verschiebbar an der Halteeinrichtung (30) angeordnet sind, wobei
- die Halteeinrichtung (30) eine erste Klemmeinrichtung (34) zur Fixierung der Führungseinrichtung (10) relativ zur Halteeinrichtung aufweist, so dass die Bewegung der Führungseinrichtung (10) relativ zur Halteeinrichtung (30) blockierbar ist,
- die Führungseinrichtung (10) und die Verschiebeeinrichtung (20) um eine Längsachse der Halteeinrichtung drehbar angeordnet sind, und
- ein Drehlager (40) vorgesehen ist, das fest mit einem Haltebolzen (42) zur ortsfesten Positionierung relativ zum zu behandelnden Organismus ausgestattet ist und in dem die Halteeinrichtung (30) mit der Führungseinrichtung (10) und der Verschiebeeinrichtung (20) um die Längsachse der Halteeinrichtung (30) drehbar sind, wobei
- eine Rastnase (43) sich im Drehlager (40) befindet, die mit Ausnehmungen (37.1) auf der Oberfläche des Lagerteils (37) zusammenwirkt,
- das Drehlager (40) einen Dorn (41) aufweist, der mit einem Lochkranz (23) der Verschiebeeinrichtung (20) zusammenwirkt und mit dem eine Verdrehung der Halteeinrichtung (30) in einem vorgeschobenen Zustand der Verschiebeeinrichtung (20) blockiert ist, und
- die im Drehlager mit einem Lagerteil (37) sitzende Halteeinrichtung (30) relativ zu dem Organismus nicht verschiebbar ist.

2. Injektionsvorrichtung nach Anspruch 1, bei der die Halteeinrichtung (30) einen Vorschub-Anschlag (31) aufweist, durch den ein Vorschub der Verschiebeeinrichtung (20) relativ zur Halteeinrichtung (30) begrenzt ist.

3. Injektionsvorrichtung nach Anspruch 1 oder 2, bei der die Halteeinrichtung (30) einen Rückzug-Anschlag (32) aufweist, durch den ein Rückzug der Führungseinrichtung (10) relativ zur Halteeinrichtung (30) begrenzt ist.

4. Injektionsvorrichtung nach einem der vorhergehenden Ansprüche, bei der die Halteeinrichtung (30) einen rohrförmigen Aufbau aufweist, wobei die Führungseinrichtung (10) innen in der Halteeinrichtung (30) und die Verschiebeeinrichtung (20) außen auf der Halteeinrichtung (30) angeordnet sind.

5. Injektionsvorrichtung nach einem der vorhergehenden Ansprüche, bei welcher der Lochkranz (23) Führungslöcher (24) aufweist, deren Anzahl und/oder Winkelverteilung in Abhängigkeit von der gewünschten geometrischen Verteilung der Injektion im Organismus gewählt ist.

6. Injektionsvorrichtung nach mindestens einem der vorhergehenden Ansprüche , bei der die erste Klemmeinrichtung eine Klemmschraube (34) mit einem Spülkanal (34.1) umfasst, durch den eine Spülflüssigkeit in die Führungseinrichtung (10) einführbar ist.

7. Injektionsvorrichtung nach mindestens einem der vorhergehenden Ansprüche, bei der die Führungseinrichtung (10) einen Kanal (12) zur Aufnahme einer bildgebenden Sonde (50) aufweist.

8. Injektionsvorrichtung nach Anspruch 7, bei der die Halteeinrichtung (30) eine zweite Klemmeinrichtung (39) zur Fixierung der Sonde (50) im Kanal (12) aufweist.

9. Injektionsvorrichtung nach mindestens einem der vorhergehenden Ansprüche, bei der die Verschiebeeinrichtung (20) eine Halterung (22) zur Aufnahme eines Injektionsreservoirs (60) aufweist.

10. Injektionsvorrichtung nach Anspruch 9, bei der die Halterung (22) zur Aufnahme des Injektionsreservoirs eine Stellschraube (23) zur Dosierung der zu injizierenden Substanz aufweist.

11. Injektionsvorrichtung nach mindestens einem der vorhergehenden Ansprüche, bei der eine Sensoreinrichtung (70) mit mindestens einem Sensor zur Erfassung der Position von mindestens einer der Führungs-, Verschiebe- und Halteeinrichtungen (10, 20, 30) aufweist.

12. Injektionsvorrichtung nach mindestens einem der vorhergehenden Ansprüche, bei der die Halteeinrichtung (30) eine Rückzugsfeder (35) aufweist, mit der die Verschiebeeinrichtung (20) von einer vorgeschobenen Betriebsposition zurückgezogen werden kann.

## Claims

1. Injection device (100) for injecting a substance into an organism (1), comprising:
- a guide device (10) with a lumen (11) in which an injection tool (21) is arranged in a displaceable manner,
- a displacement device (20) to which the injection tool (21) is connected, and
- a holder device (30) that forms a support for the guide device (10) and for the displacement device (20), the guide and displacement devices (10, 20) being arranged at the holder device (30) such that they can be displaced relative to one another and relative to the holder device (30), wherein
- the holder device (30) has a first clamping device (34) for fixing the guide device (10) relative to the holder device, so that the movement of the guide device (10) relative to the holder device (30) can be blocked,
- the guide device (10) and the displacement device (20) are arranged such that they can be rotated about a longitudinal axis of the holder device, and
- a rotary bearing (40) is provided, which is fixedly provided with a retaining bolt (42) for stationary positioning relative to the organism to be treated and in which the holder device (30) with the guide device (10) and the displacement device (20) can be rotated about the longitudinal axis of the holder device (30), wherein
- a latching protrusion (43) is arranged in the rotary bearing (40) and cooperates with recesses (37.1) on the surface of the bearing part (37),
- the rotary bearing (40) has a mandrel (41) which cooperates with a perforated ring (23) of the displacement device (20) and which blocks a rotation of the holder device (30) in a pushed-forward state of the displacement device (20), and
- the holder device (30) placed in the rotary bearing with a bearing part (37) cannot be displaced relative to the organism.

2. The injection device according to claim 1, in which the holder device (30) has a forward stop (31) which limits a forward movement of the displacement device (20) relative to the holder device (30).

3. The injection device according to claim 1 or 2, in which the holder device (30) has a backward stop (32) which limits a backward movement of the guide device (10) relative to the holder device (30).

4. The injection device according to one of the preceding claims, in which the holder device (30) has a tubular structure, the guide device (10) being arranged internally within the holder device (30) and the displacement device (20) being arranged externally on the holder device (30).

5. The injection device according to one of the preceding claims, in which the perforated ring (23) has guide holes (24), the number and/or angular distribution of which is selected as a function of the desired geometric distribution of the injection into the organism.

6. The injection device according to at least one of the preceding claims, in which the first clamping device comprises a clamping screw (34) with a flushing channel (34.1), through which a flushing fluid can be introduced into the guide device (10).

7. The injection device according to at least one of the preceding claims, in which the guide device (10) has a channel (12) for accommodating an imaging probe (50).

8. The injection device according to claim 7, in which the holder device (30) has a second clamping device (39) for fixing the probe (50) in the channel (12).

9. The injection device according to at least one of the preceding claims, in which the displacement device (20) has a mount (22) for holding an injection reservoir (60).

10. The injection device according to claim 9, in which the mount (22) for holding the injection reservoir has an adjusting screw (23) for metering the substance that is to be injected.

11. The injection device according to at least one of the preceding claims, in which a sensor device (70) is provided, which comprises at least one sensor for detecting the position of at least one of the guide, displacement and holder devices (10, 20, 30).

12. The injection device according to at least one of the preceding claims, in which the holder device (30) has a return spring (35), by means of which the displacement device (20) can be returned from a pushed-forward operating position.

## Revendications

1. Dispositif d'injection (100) servant à injecter une substance dans un organisme (1), comprenant :
- un système de guidage (10) pourvu d'un lumen (11), dans lequel un outil d'injection (21) est disposé de manière à pouvoir être coulissé,
- un système de coulissement (20), auquel l'outil d'injection (21) est relié, et
- un système de maintien (30), qui forme un support pour le système de guidage (10) et pour le système de coulissement (20), dans lequel les systèmes de guidage et de coulissement (10, 20) sont disposés au niveau du système de maintien (30) de manière à pouvoir être coulissés l'un par rapport à l'autre et par rapport au système de maintien (30), dans lequel
- le système de maintien (30) présente un premier système de serrage (34) servant à fixer le système de guidage (10) par rapport au système de maintien de sorte que le déplacement du système de guidage (10) par rapport au système de maintien (30) peut être bloqué,
- le système de guidage (10) et le système de coulissement (20) sont disposés de manière à pouvoir tourner autour d'un axe longitudinal du système de maintien, et
- un palier rotatif (40) est prévu, lequel est équipé d'un boulon de maintien (42) servant au positionnement de manière stationnaire par rapport à l'organisme à traiter et dans lequel le système de maintien (30) pourvu du système de guidage (10) et du système de coulissement (20) peut être tourné autour de l'axe longitudinal du système de maintien (30), dans lequel
- un ergot d'arrêt (43) se trouve dans le palier rotatif (40), lequel coopère avec des évidements (37.1) sur la surface de la partie de palier (37),
- le palier rotatif (40) présente un mandrin (41), qui coopère avec une couronne perforée (23) du système de coulissement (20) et à l'aide duquel une torsion du système de maintien (30) est bloquée dans un état avancé du système de coulissement (20), et
- le système de maintien (30) reposant dans le palier rotatif avec une partie de palier (37) ne peut pas être coulissé par rapport à l'organisme.

2. Dispositif d'injection selon la revendication 1, dans le cadre duquel le système de maintien (30) présente une butée d'avancée (31), par laquelle une avancée du système de coulissement (20) est limitée par rapport au système de maintien (30).

3. Dispositif d'injection selon la revendication 1 ou 2, dans le cadre duquel le système de maintien (30) présente une butée de retour (32), par laquelle un retour du système de guidage (10) est limité par rapport au système de maintien (30).

4. Dispositif d'injection selon l'une quelconque des revendications précédentes, dans le cadre duquel le système de maintien (30) présente une structure de forme tubulaire, dans lequel le système de guidage (10) est disposé à l'intérieur du système de maintien (30) et que le système de coulissement (20) est disposé à l'extérieur sur le système de maintien (30).

5. Dispositif d'injection selon l'une quelconque des revendications précédentes, dans le cadre duquel la couronne perforée (23) présente des trous de guidage (24), dont le nombre et/ou la répartition angulaire sont choisis en fonction de la répartition géométrique souhaitée de l'injection dans l'organisme.

6. Dispositif d'injection selon au moins l'une quelconque des revendications précédentes, dans le cadre duquel le premier système de serrage comprend une vis de serrage (34) pourvue d'un canal de rinçage (34.1), par lequel un liquide de rinçage peut être introduit dans le système de guidage (10).

7. Dispositif d'injection selon au moins l'une quelconque des revendications précédentes, dans le cadre duquel le système de guidage (10) présente un canal (12) servant à recevoir une sonde (50) d'imagerie.

8. Dispositif d'injection selon la revendication 7, dans le cadre duquel le système de maintien (30) présente un deuxième système de serrage (39) servant à la fixation de la sonde (50) dans le canal (12).

9. Dispositif d'injection selon au moins l'une quelconque des revendications précédentes, dans le cadre duquel le système de coulissement (20) présente un support (22) servant à recevoir un réservoir d'injection (60).

10. Dispositif d'injection selon la revendication 9, dans le cadre duquel le support (22) servant à recevoir le réservoir d'injection présente une vis de réglage (23) servant à doser la substance à injecter.

11. Dispositif d'injection selon au moins l'une quelconque des revendications précédentes, dans le cadre duquel un système de capteur (70) présente au moins un capteur servant à détecter la position d'au moins des systèmes de guidage, de coulissement et de maintien (10, 20, 30).

12. Dispositif d'injection selon au moins l'une quelconque des revendications précédentes, dans le cadre duquel le système de maintien (30) présente un ressort de rappel (35), à l'aide duquel le système de coulissement (20) peut être retiré d'une position de fonctionnement avancée.
